# EUROPÄISCHE PATENTSCHRIFT

(11) **EP 2 247 926 B1**
(45) Veröffentlichungstag und Bekanntmachung des Hinweises auf die Patenterteilung: **25.04.2012**
(21) Anmeldenummer: 09715403.3
(22) Anmeldetag: 24.02.2009
(51) Int. Cl.: G01D 18/00

(54) **VERFAHREN ZUM KALIBRIEREN EINES SENSORS INNERHALB EINER KAMMER, SENSOR, DISPOSABLE UND BEHANDLUNGSVORRICHTUNG MIT EINEM SOLCHEN SENSOR**
METHOD FOR CALIBRATING A SENSOR WITHIN A CHAMBER, SENSOR, DISPOSABLE DEVICE, AND TREATMENT DEVICE HAVING SUCH A SENSOR
PROCÉDÉ D'ÉTALONNAGE D'UN CAPTEUR DANS UNE CHAMBRE, CAPTEUR, ARTICLE JETABLE ET DISPOSITIF DE TRAITEMENT COMPORTANT UN TEL CAPTEUR

(30) Priorität: 25.02.2008 DE 102008010948
(43) Veröffentlichungstag der Anmeldung: 10.11.2010
(73) Patentinhaber: Fresenius Medical Care Deutschland GmbH, 61352 Bad Homburg (DE)
(72) Erfinder: HEIDE, Alexander, 65817 Eppstein (DE); METZNER, Klaus, 61381 Friedrichsdorf (DE)
(74) Vertreter: Bobbert & Partner
(86) Internationale Anmeldenummer: PCT/EP2009/001306
(87) Internationale Veröffentlichungsnummer: WO 2009/106293

(56) Entgegenhaltungen:
- US-A- 5 143 617
- US-B1- 6 841 401
- US-B1- 6 850 859
- US-B2- 6 695 806

## Beschreibung

Die vorliegende Erfindung betrifft ein Verfahren zum Kalibrieren eines Sensors gemäß dem Oberbegriff des Anspruchs 1. Sie betrifft ferner einen Sensor gemäß dem Oberbegriff des Anspruchs 9, ein Disposable gemäß dem Oberbegriff des Anspruchs 11 sowie eine Behandlungsvorrichtung gemäß dem Oberbegriff des Anspruchs 12.

Bei einer Reihe von aus der Praxis bekannten Verfahren wird wiederholt der Wert eines oder mehrerer Parameter zur Kontrolle und Steuerung des Verfahrens gemessen. Solche Verfahren sind aus dem industriellen Bereich ebenso wie aus dem medizinischen Bereich bekannt. So werden im medizinischen Bereich bspw. bei der Dialysebehandlung eines Patienten unter Einsatz eines extrakorporalen Blutkreislaufs der venöse und der arterielle Druck im extrakorporalen Blutkreislauf gemessen und das Verfahren entsprechend den gemessenen Werten gesteuert.

Zum Ermitteln des Drucks im extrakorporalen Blutkreislauf kann dieser mit "Stichleitungen" (Druckableitungen) versehen sein, um mittels einer Gassäule den Druck im extrakorporalen Kreislauf an einen Drucksensor weiterzuleiten. Diese Stichleitungen stellen "offene" Stellen des extrakorporalen Kreislaufs dar und müssen aus hygienischen Gründen u. a. mit sterilen Filtern ("transducer protector") geschützt werden. Ein Versagen dieser Filter bzw. Schutzeinrichtungen allgemein kann den Patienten der Gefahr einer Crosskontamination aussetzen.

Ein weiteres bekanntes Verfahren zum Messen des extrakorporalen Drucks erfolgt mittels sogenannter "Druckdome". Hierbei bleibt das Schlauchsystem geschlossen, die Druckwerte werden über eine undurchlässige Membran vom Blut in die Maschine übertragen. Zu diesem Zweck ist ein wiederverwendbarer Drucksensor im extrakorporalen Kreislauf angeordnet. Der Sensor muss dabei über einen vergleichsweise langen Zeitraum von bspw. zwei Jahren und bis zu zehn Jahren erheblichen Anforderungen an Hygiene, Messwert-Drift usw. gerecht werden.

Daher sind im Stand der Technik auch Drucksensoren, die drahtgebunden oder auch mittels Funk Messwerte übertragen, für Einwegartikel bzw. Disposables bekannt. Sie befinden sich beim Beispiel des extrakorporalen Blutkreislaufs innerhalb des Einweg-Schlauchsystems und werden nach der Behandlung gemeinsam mit dem Disposable entsorgt. Solche Disposable-Drucksensoren erfordern jedoch eine für jeden Sensor einzeln vorzunehmende Kalibrierung, was ihre Herstellung teuer gestaltet. Ferner müssen vom Lieferanten der Sensoren Kalibrierdaten in separaten Datenspeichern mit den Sensoren mitgeliefert werden, wie dies bspw. im US-Patent 6,695,806 B2 der CHF Solutions beschrieben ist. Es sind insbesondere die hohen Kosten, die durch die zur Kalibrierung erforderliche Kontaktierung jedes einzelnen Sensors entstehen, welche den Einsatz dieser Sensoren bei großen und insbesondere sehr großen Stückzahlen - in welchen die meisten Einwegartikel hergestellt und verbraucht werden - nicht rechtfertigen.

Aus der US 6 841 401 B1 ist ein Kalibrierungssystem für eine integrierte Schaltungsvorrichtung sowie ein Verfahren zur Fehlerkorrektur einer integrierten Schaltungsvorrichtung bekannt.

Aus der US 6 695 806 B2 ist ein integriertes Einmalset zur Dialyse- oder Ultrafiltrationsbehandlung von Blut bekannt.

Aus der US 6 850 859 B1 ist ein Driftkompensationssystem zum Kompensieren eines Sensordrifts bekannt.

Die Aufgabe der vorliegenden Erfindung besteht darin, ein weiteres Verfahren zum Kalibrieren eines Sensors vorzuschlagen. Ferner soll ein mittels des erfindungsgemäßen Verfahrens kalibrierter Sensor, ein einen solchen Sensor aufweisendes Disposable (Einweg- bzw. Wegwerfartikel), und eine Behandlungsvorrichtung mit einem solchen Sensor und/ oder einem solchen Disposable angegeben werden.

Die erfindungsgemäße Aufgabe wird gelöst durch ein Verfahren mit den Merkmalen des Anspruchs 1.

So wird erfindungsgemäß ein Verfahren zum Kalibrieren wenigstens eines Sensors, insbesondere eines RFID-Sensors (Radio Frequency Identification), vorgeschlagen, wobei der Sensor wenigstens eine Speichereinrichtung zum Speichern wenigstens eines Wertes wenigstens eines Parameters aufweist. Der Parameter kann hierbei physikalischer, chemischer biologischer wie Druck, Temperatur, Leitfähigkeit, Konzentration oder dgl., oder jeder anderen Art sein.

Der Sensor kann ein Interface für eine RFID-Komponente mit r/w-Speicher (read/write) aufweisen, welcher die Kalibrierung des Sensors bereits während dessen Produktionsprozesses erlaubt; er kann zudem Informationen wie Produktidentifikationsdaten für eine spätere Prüfung einer mit dem Sensor verbundenen Einrichtung, insbesondere medizinischen Einrichtung wie einem Disposable oder einem Mehrweg-Gegenstand auf Eignung für das gewählte Behandlungsverfahren speichern. Unter einem Speicher wird erfindungsgemäß auch eine Mehrzahl von Speichern, welche getrennt voneinander vorliegen können, verstanden.

Das Verfahren umfasst den Schritt des Anordnens des Sensors in einer Kammer, wobei in der Kammer zu wenigstens einem Zeitpunkt, zu welchem der Sensor innerhalb der Kammer angeordnet ist, ein bekannter Wert des Parameters herrscht.

Der Sensor ist vorzugsweise geeignet, einen oder mehrere Werte eines oder mehrerer Parameter zu speichern, welche während dessen Anwesenheit in der Kammer in dieser herrschen.

Unter einer "Kammer" wird erfindungsgemäß ein umschlossener Raum verstanden. Ebenso wird unter "Kammer" erfindungsgemäß auch jeder halboffene oder offene Ort verstanden, in welchem zu wenigstens einem Zeitpunkt, zu welchem sich der Sensor an diesem Ort befindet, der Parameter, auf welchen der Sensor kalibriert werden soll, mit einem Wert vorliegt, welcher mit einer für eine Kalibrierung des Sensors ausreichenden Genauigkeit bekannt ist.

Unter dem "Wert" des Parameters wird erfindungsgemäß eine veränderliche Ausprägung des Parameters selbst verstanden. So ist bspw. in der Angabe "3 bar" die Zahl 3 als Wert im Sinne der Erfindung zu verstehen, wohingegen die Dimension "bar" den Parameter selbst - in diesem Fall der Druck - angibt. Wenn im Folgenden von einem Parameter die Rede ist, so kann sich dies stets auch auf eine Mehrzahl von Parametern erstrecken, wo immer die Ausführungen zum Parameter für den Fachmann erkennbar ebenfalls zutreffen.

Bei der Durchführung des erfindungsgemäßen Kalibrierverfahrens wird der in der Kammer herrschende Wert des Parameters zu einem oder zu mehreren Zeitpunkten vom in der Kammer angeordneten Sensor ermittelt. Dies kann bspw. durch einen Messvorgang oder durch ein anderes Bestimmungsverfahren oder Kombinationen hiervon erfolgen. Zu dem wenigstens einen Zeitpunkt, zu welchem der innerhalb der Kammer vorliegende Parameterwert vom Sensor gemessen werden soll, kann dem Sensor ein von diesem innerhalb der Kammer empfangbares Signal zum Ermitteln und Speichern des Parameterwertes übermittelt werden. Dabei kann das an den Sensor abgegebene Signal zugleich Informationen über den bekannten, tatsächlich innerhalb der Kammer herrschenden Parameterwert enthalten. Der vom Sensor gemessene oder ermittelte Parameterwert kann sofort oder auch zu einem späteren Zeitpunkt zum Erstellen einer Kalibrierkurve verwendet werden. Die Kalibrierkurve kann im Sensor abgespeichert werden.

Ein mittels des erfindungsgemäßen Verfahrens zum Kalibrieren eines Sensors erzielbarer Vorteil besteht darin, dass durch das Anordnen des Sensors in einer Kammer - im Sinne eines wie auch immer gestalteten Einbringens in die Kammer - mit wenigstens zu einem Zeitpunkt bekannten Kammerinnenraumbedingungen eine zeitaufwändige, apparatgebundene Kontaktierung des Sensors zu seiner Kalibrierung vorteilhaft entfallen kann. Vielmehr kann bspw. ein ohnehin erfolgendes Durchlaufen des Sensors durch die Kammer - bspw. bei dessen Herstellung- vorteilhaft zur Kalibrierung "mitbenutzt" werden. Das erfindungsgemäße Verfahren zeichnet sich somit durch ein vergleichsweise wenig aufwändiges und kostengünstiges Kalibrieren des Sensors aus.

Weitere Vorteile bestehen darin, dass anders als beim bekannten Verwenden von offenen Stichleitungen oder Druckdomen keine maschinenseitigen Drucksensoren mehr erforderlich sind und keine Kosten für Wartung und dergleichen anfallen. Zudem erfordert der Einsatz von erfindungsgemäß hergestellten Sensoren, insbesondere im medizinischen Bereich, auch keine Verwendung mehr von "transducer protectors"; dennoch ist eine Gefahr von Crosskontaminationen über die Druckableitungen nicht mehr gegeben: Der Einsatz von erfindungsgemäß hergestellten Sensoren erlaubt die Messung des Parameters innerhalb eines luftfreien, geschlossenen Systems. Im medizinischen Bereich kann eine Messung vorteilhaft ohne Kontakt zwischen Blut und Umgebung bzw. Luft, bei minimaler Kontaktfläche zwischen Sensor und Blut, erfolgen.

So wird vorgeschlagen, das Verfahren mit Hilfe einer Sterilisationskammer, in welcher der Sensor angeordnet wird, durchzuführen. In solchen Sterilisationskammern wird üblicherweise u. a. unter Einsatz von Dampf, ETO, Ebeam, Plasma, Strahlung oder dgl. sterilisiert.

Sterilisationskammern zeichnen sich i. d. R. dadurch aus, dass - insbesondere bei industrieller Sterilisation - Prozesse mit kontrollierten und exakt bekannten Prozessbedingungen ablaufen. Beim Durchführen des erfindungsgemäßen Verfahrens unter Einbeziehung einer Sterilisationskammer kann daher vorteilhaft ein ohnehin stattfindender Sterilisationsprozess innerhalb einer Sterilisationskammer genutzt werden, um den Sensor ohne nennenswerten zusätzlichen Aufwand wie oben beschrieben zu kalibrieren. Es müssen nicht eigens geeignete Umgebungsbedingungen zum Kalibrieren des Sensors geschaffen werden.

Das erfindungsgemäße Verfahren dieser Ausführungsform eignet sich besonders gut zum Kalibrieren von Sensoren, welche zu ihrer Verwendung steril sein müssen. Der ohnehin ablaufende Sterilisierungsprozess kann zugleich zum Kalibrieren des Sensors "mitverwendet" werden.

Vorteilhafte Weiterbildungen des Erfindungsgegenstandes sind jeweils Gegenstand der Unteransprüche.

Eine bevorzugte Ausführungsform des erfindungsgemäßen Verfahrens sieht vor, dass in der Speichereinrichtung des Sensors zusätzlich zu dem oben diskutierten wenigstens einen Parameterwert auch eine Zeitdauer gespeichert wird. Diese Zeitdauer kann bei entsprechender Signalgabe bspw. angeben, wie lange der Sensor bestimmten Bedingungen ausgesetzt gewesen ist. Das Auslesen der in der Speichereinrichtung des Sensors gespeicherten Informationen kann aufgrund der Zeitangabe somit zu zusätzlichen Aussagen führen, welche vorteilhaft für Informationen über innerhalb der Kammer ablaufende Prozesse genutzt werden können.

Beim Beispiel der Sterilisationskammer als Kammer im Sinne des erfindungsgemäßen Verfahrens kann die gespeicherte Zeitdauer bei entsprechender Steuerung des Sensors u. a. eine Aussage darüber erlauben, ob der Sensor - und etwa ein mit ihm verbundenes Element wie das weiter unten beschriebene Disposable - ausreichend lange einem Sterilisationsprozess ausgesetzt gewesen ist. Soll die Sterilisation bspw. bei ca. 120°C und dem im Falle einer Dampfsterilisierung zugehörigen Druck stattfinden, und muss der Sensor dieser Temperatur für eine sicher gewährleistete Sterilität für eine bestimmte Mindestzeitdauer ausgesetzt sein, so kann anhand der gespeicherten Zeitdauer bzw. anhand gespeicherter Daten, welche einen Rückschluss auf die Zeitdauer erlauben, zu einem späteren Zeitpunkt überprüft werden, ob der Sensor - und ein ggf. mit ihm verbundenes Disposable - als steril gelten darf oder nicht. Ist ein Sensor nicht ausreichend lange auf der vorgeschriebenen Temperatur gehalten worden, so kann er und ein mit ihm verbundenes Element wie ein Disposable aufgrund der gespeicherten Zeitangabe als nicht steril identifiziert und verworfen werden. Auf diese Weise erhält jeder nach dem erfindungsgemäßen Verfahren dieser Ausführungsform kalibrierte Sensor - und mit ihm verbundene Elemente - eine individuelle Sterilitätsbescheinigung.

Ferner erlaubt das erfindungsgemäße Verfahren in der vorliegenden Ausführungsform eine zusätzliche Kontrolle der Bedingungen, welche in der Kammer während der Anwesenheit des Sensors dort tatsächlich geherrscht haben.

Diese Ausführungen, welche am Beispiel der Sterilisationskammer gemacht wurden, lassen sich für den Fachmann erkennbar auch auf andere Kammern als die Sterilisationskammer übertragen. Solche Kammern können Druckkammern und dergleichen sein. Hierzu zählen auch Kammern, in welchen keine sterilen Bedingungen geschaffen sondern lediglich die Keimzahl verringert werden soll (keimarm).

Gemäß einer wiederum weiter bevorzugten Ausführungsform des erfindungsgemäßen Verfahrens wird eine Mehrzahl oder Vielzahl von Sensoren, insbesondere ein Batch oder eine Charge von Sensoren, gemeinsam in der Kammer angeordnet, so dass sie zu wenigstens einem Zeitpunkt gleichzeitig dort angeordnet sind. Die Mehrzahl von Sensoren wird auf diese Weise zeitgleich und wiederum wie oben beschrieben ohne zusätzlichen apparativen Aufwand kalibriert. Signale, die von außerhalb der Kammer an die Sensoren gerichtet sind, können von jedem Sensor aus der Mehrzahl von Sensoren empfangen werden und zu einer entsprechenden Reaktion aller Sensoren führen. Daher ist es erfindungsgemäß vorteilhaft möglich, ohne körperlichen Kontakt, wie mittels einer Leitungsverbindung, mit einer Mehrzahl oder Vielzahl von Sensoren zu kommunizieren, was den Aufwand des Kalibrierens insgesamt äußerst gering hält.

Ist für jeden der Sensoren eine Ablageposition in der Kammer bekannt, was insbesondere beim automatischen Beschicken der Kammer der Fall sein kann, so können die in den einzelnen Speichereinrichtungen der Sensoren jeweils gespeicherten Informationen über die Bedingungen innerhalb der Kammer die Kontrolle der in der Kammer ablaufenden Prozesse, wie z. B. eines Sterilisierungsvorganges, ermöglichen oder unterstützen. Ist ferner die Ablageposition des Sensors in der Kammer bekannt, so kann aus der Ablageposition zudem ein exaktes Druck- und/ oder Temperaturprofil (sowie auch andere Profile) für die Kammer ermittelt werden, was einen wichtigen und kostengünstigen Beitrag zu aufwändigen In-Prozess-Kontrollen und Validierungen darstellen kann.

In einer wiederum weiter bevorzugten Ausführungsform ist der Sensor mit einem Mehrfachartikel oder einem Disposable bzw. Einmalartikel verbunden - d.h. diesem auf beliebige Art zugeordnet, unabhängig davon, ob eine form - und /oder kraftschlüssige Verbindung zwischen Sensor und Disposable vorliegt - und liegt mit diesem gemeinsam in der Kammer vor. Die Behandlung, die dem Sensor in der Kammer widerfährt, widerfährt somit zugleich auch dem Disposable, und umgekehrt. Dies ist bspw. beim Sterilisieren der Disposables in einer Sterilisationskammer von besonderem Interesse, da auf diese Weise neben dem Disposable zugleich auch der Sensor, welcher später ggf. mit Körperflüssigkeiten in Kontakt gebracht wird, sterilisiert wird.

Zudem ist der Sensor bei dieser Ausführungsform des erfindungsgemäßen Verfahrens vorzugsweise denselben Kammerbedingungen ausgesetzt wie der Mehrfachartikel oder das Disposable. Der Sensor kann dabei vorzugsweise Werte von Parametern speichern, welchen auch der Mehrfachartikel oder das Disposable ausgesetzt ist.

Dieses Verfahren weist ferner die oben bereits beschriebenen Vorteile - auf welche zur Vermeidung von Wiederholungen an dieser Stelle explizit verwiesen wird - auf, nämlich das einfache, apparativ wenig aufwändige und kostengünstige Kalibrieren des Sensors eines Disposables während eines ohnehin für das Disposable zwingend erforderlichen Sterilisationsschrittes in einer Sterilisationskammer.

Es wird darauf hingewiesen, dass sich diese Vorteile nicht auf den Sterilisationsprozess in einer Sterilisationskammer beschränken. Sie sind vielmehr auch beim gemeinsamen Anordnen des Sensors und des Disposables in anderen Kammern als einer Sterilisationskammer erzielbar.

Die vorliegende Erfindung betrifft in einer wiederum weiter bevorzugten Ausführungsform ein Verfahren zum Kalibrieren wenigstens eines Sensors, insbesondere eines RFID-Sensors, welcher eine Speichereinrichtung zum Speichern wenigstens eines Wertes wenigstens eines, insbesondere physikalischen oder chemischen oder biologischen Parameters aufweist, mit einem Anordnen des Sensors in einer Kammer, in welcher zu wenigstens einem Zeitpunkt ein bekannter Wert des Parameters herrscht, wobei der Sensor mit einem einer, insbesondere medizinischen Einrichtung - einem Disposable oder einem mehrfach verwendbaren Gegentand - verbunden in der Kammer angeordnet wird; und mittels der Speichereinrichtung ein bekannter, in der Kammer herrschender Wert des Parameters gespeichert wird. Das erfindungsgemäße Verfahren dieser Ausführungsform kann vorzugsweise mit beliebigen, hierin ebenfalls offenbarten Verfahrensmerkmalen kombiniert werden, soweit der Fachmann eine Kombinierbarkeit erkennen kann. Derartige Kombinationen sind hiermit ausdrücklichen von der Erfindung mit umfasst.

Die erfindungsgemäße Aufgabe wird auch gelöst durch einen Sensor mit den Merkmalen des Anspruchs 9, durch ein Disposable mit den Merkmalen des Anspruchs 11 sowie durch eine Behandlungsvorrichtung, insbesondere eine Dialysevorrichtung wie z. B. eine Hämodialyse, mit den Merkmalen des Anspruchs 12. Da alle oben im Zusammenhang mit dem erfindungsgemäßen Verfahren diskutierten Vorteile ungeschmälert auch mit dem erfindungsgemäßen Sensor, dem Disposable und der Behandlungsvorrichtung erzielbar sind, wird zur Vermeidung von Wiederholungen auf die obenstehende Diskussion der neben anderen erzielbaren Vorteile ausdrücklich verwiesen. Bei dem Disposable kann es sich um jeden Einwegartikel handeln, sofern er die Merkmale des Anspruchs 11 aufweist, insbesondere um medizinische Disposables, vor allem Dialysedisposables wie extrakorporale Blutschlauchsätze und dergleichen. Ein zusätzlicher Vorteil, welche ein erfindungsgemäßes Disposable bietet, ist dass die Lebensdauer seines Sensors aufgrund des einmaligen Gebrauchs des Disposables wenige Stunden bis wenige Tage i. a. R. nicht zu überschreiten braucht. Entsprechend einfach und kostengünstig können die Sensoren hergestellt werden.

Die vorliegende Erfindung wird im Folgenden anhand der beigefügten Zeichnung detaillierter erläutert. In der Zeichnung gilt:
- Fig. 1: zeigt den Ablauf einer exemplarischen Ausführungsform des erfindungsgemäßen Verfahrens;
- Fig.2: zeigt das Vorgehen bei einem Plausibilitätstest und eine Steigungsanpassung im Rahmen einer exemplarischen Ausführungsform des erfindungsgemäßen Verfahrens; und
- Fig. 3: zeigt die Veränderung von Druck und Temperatur in einer Kammer im Zeitverlauf.

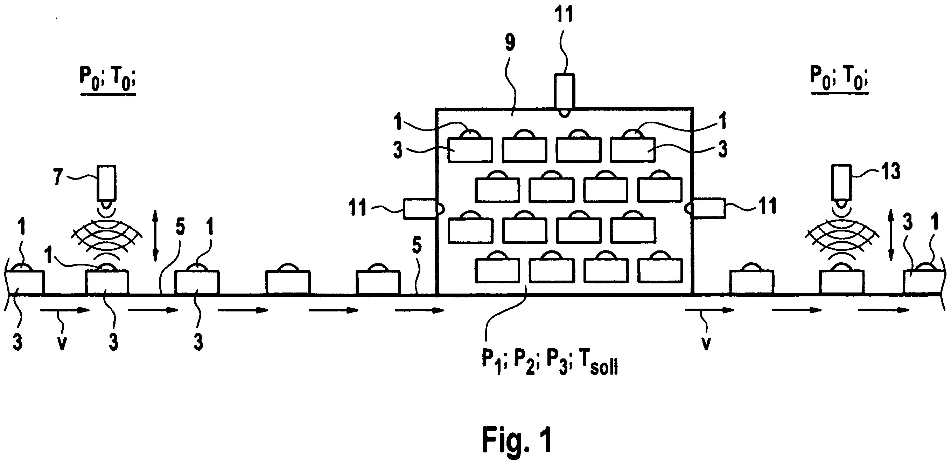

Fig. 1 zeigt am Beispiel von schematisch vereinfachten dampfsterilisierten Blutschlauchsets als Beispiel für Disposables 3, wobei jeder Blutschlauchsatz 3 mindestens einen RFID-Sensor 1 aufweist, einen möglichen Ablauf des erfindungsgemäßen Verfahrens. Die im Ausführungsbeispiel der in Fig. 1 dargestellten Schritte des erfindungsgemäßen Verfahrens schließen sich an ein bekanntes Produktionsverfahren für die Schlauchsets 3 an. Dabei liegen die mit den RFID-Sensoren 1 bestückten Disposables 3 vereinzelt auf einem Förderband 5. Die Bewegungsrichtung des Förderbands 5 ist in Fig. 1 mit den Pfeilen v angegeben. Die Sensoren 1 werden am linken Rand der Fig. 1 mittels einer RFID-Readereinheit 7 in einem ersten Funktionstest abgefragt. Dabei kann festgestellt werden, ob das Disposable 3 überhaupt mit einem Sensor 1 ausgestattet ist, und ob dieser Sensor 1 seine Kennung beim Anfunken korrekt überträgt.

Ferner kann als Plausibilitätstest überprüft werden, ob der Sensor 1 die im Verfahrensschritt des linken Randes der Fig. 1 vorliegenden normalen Umgebungstemperaturen T₀ und P₀ korrekt innerhalb eines Toleranzbereichs angibt. Sollte dieser erste Plausibilitätstest und/ oder der Funktionstest von einem Disposable 3 bzw. dessen Sensor 1 nicht bestanden werden, so kann das entsprechende Disposable 3 samt seinem Sensor 1 als Ausschuss verworfen werden. Die Speicher der Sensoren 1 der nicht als Ausschuss erkannten Disposables 3 können mit Produktionsdaten, welche sowohl den Sensor 1 als auch das Disposable 3 betreffen können, sowie Kalibrierdaten beschrieben und serialisiert werden. Durch die Speicherung von Produktionsdaten lässt das einzelne Produkt später bei Bedarf leicht verfolgen.

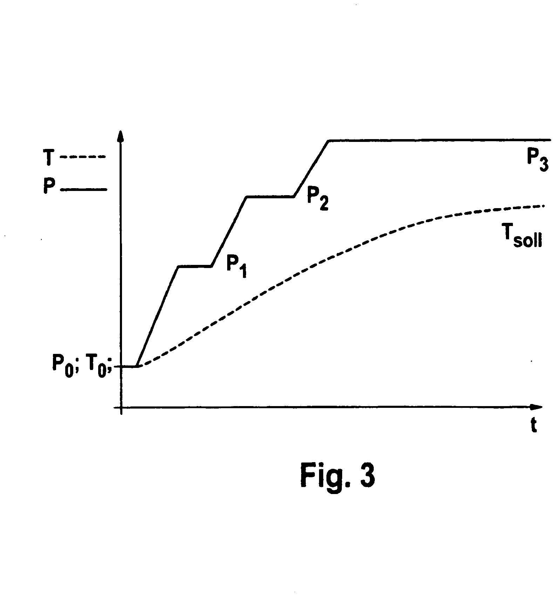

Die nicht als Ausschuss gewerteten Disposables 3 werden auf dem Förderband 5 in eine mit Disposables 3 zu beschickende Kammer 9 weitertransportiert. Die Kammer 9 ist im Beispiel der Fig. 1 als eine Sterilisierkammer ausgestaltet, in welcher ein Sterilisationsprozess abläuft. Sofern die Kammer automatisiert befüllt wird, kann die Ablageposition eines jeden Disposables 3 innerhalb der Kammer 9 auf einfache Weise nachvollzogen werden. Beim Sterilisationsprozess der Fig. 1 werden der Druck und die Temperatur (diese bis auf ca. 120°C) innerhalb der Kammer 9 gesteigert, wie dies in der unten diskutierten Fig. 3 erläutert ist. Zu beliebigen Zeitpunkten wird über eine oder mehrere RFID-Sendeantennen 11 ein - vorzugsweise für alle Sensoren 1 gleiches - Signal abgegeben. Das Signal veranlasst die Speicherung der jeweiligen momentanen, vom jeweiligen Sensor 1 gemessenen Druckwerte und ggf. auch Temperaturwerte im Speicher der jeweiligen Sensoren 1. Die korrespondierenden Referenzdruckwerte, d. h. die Druckwerte und ggf. auch die Temperaturwerte, die innerhalb der Kammer 9 zum jeweiligen Zeitpunkt tatsächlich geherrscht haben, können sofort ebenfalls mittels Signal in den Sensorspeicher übertragen werden. Dies kann jedoch auch zu einem späteren Zeitpunkt erfolgen, sogar dann noch, wenn die Sensoren 1 die Kammer 9 bereits verlassen haben. Die Abgabe eines solchen Signals an die innerhalb der Kammer 9 vorliegenden Sensoren 1 durch die RFID-Sendeantennen 11 kann beliebig oft wiederholt werden. Zum Erstellen einer linearen Kalibrierkurve ist bereits ein einziger Messzeitpunkt während des Kammeraufenthalts ausreichend, wenn vor oder nach dem Kammeraufenthalt ein zweiter Messzeitpunkt (Ermittlung des Nullwerts) vorgesehen wird. Dies kann bspw. der maximale Druck in einer stationären Phase des Sterilisationsvorgangs innerhalb der Kammer 9 sein. Daher kann das erfindungsgemäße Verfahren auch ohne die Abgabe eines Signals durch die RFID-Sendeantennen 11 durchgeführt werden.

Nachdem die Disposables 3 die Kammer 9 nach Beendigung des Sterilisationsprozesses verlassen haben und wiederum auf dem Förderband 5 abgelegt wurden, durchlaufen sie erneut einzeln eine RFID-Readereinheit 13. Hierbei wird wiederum ein Plausibilitätstest durchgeführt, welcher die Überprüfung umfassen kann, ob sich der RFID-Sensor 1 eines jeden Disposables 3 ordnungsgemäß meldet. Ferner kann erneut überprüft werden, ob der angezeigte Temperatur- und/ oder Druckwert, welcher vom Sensor 1 nach Verlassen der Kammer 9 gemessen wird, den tatsächlichen Umgebungsbedingungen T₀ und P₀ entspricht. Weitere Funktions- und/ oder Plausibilitätsüberprüfungen sind ebenfalls möglich.

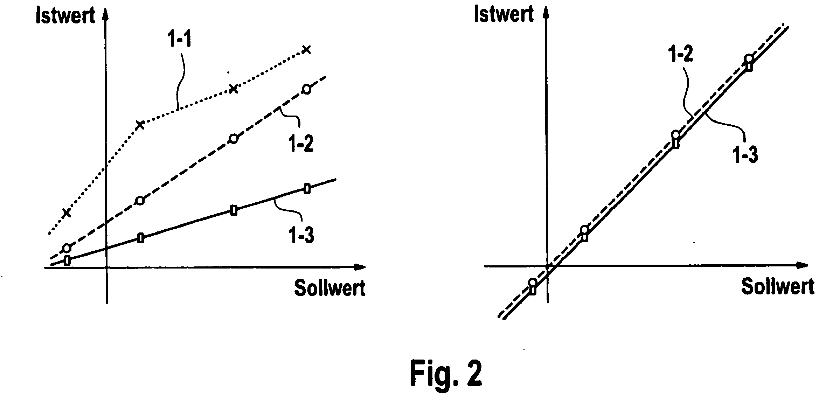

Disposables 3, deren Sensoren 1 einen dieser Tests nicht bestehen, werden wiederum als Ausschuss behandelt. Bei den verbleibenden, nicht als Ausschuss erkannten Disposables 3 wird aus den in der Speichereinheit oder den Speichereinheiten ihrer Sensoren 1 gespeicherten (Kalibrier-)Werten eine oder mehrere Kalibrierkurve(n) errechnet und im Speicher des Sensors 1 abgelegt, wie dies aus der Fig. 2 hervorgeht. Bei diesem Produktionsschritt können ferner Daten zur Sterilisierung sowie Verfallsdaten und dergleichen in den Speicher der Sensoren 1 eingeschrieben werden. Der Sterilisationsprozess innerhalb der Kammer 9 kann bei bekannter Ablageposition der einzelnen Sensoren 1 innerhalb der Kammer 9 durch Erstellen eines exakten Temperaturprofils innerhalb der Kammer 9 überprüft werden.

Fig. 2 zeigt in ihrer linken Darstellung die von drei Sensoren 1-1, 1-2 und 1-3 gemessenen Druckwerte, mittels welcher jeweils eine Kalibrierkurve errechnet und im Speicher des jeweiligen Sensors abgelegt werden kann. Wie der linken Darstellung der Fig. 2 zu entnehmen ist, liegen die gemessenen Druckwerte des Sensors 1-1 nicht auf einer Geraden, weshalb dieser Sensor 1-1 gemeinsam mit dem zugehörigen Disposable als Ausschuss verworfen wird. Für die nicht als Ausschuss erkannten Sensoren 1-2 und 1-3 werden zu ihrer Kalibrierung korrigierte Steigungsverläufe festgelegt, wie dies der rechten Darstellung der Fig. 2 zu entnehmen ist. Die Verläufe werden gespeichert.

Fig. 3 zeigt den im Zusammenhang mit Fig. 1 bereits diskutierten stufenweisen Druckanstieg von P₀ auf P₁, von P₁ auf P₂ und von P₂ auf P₃ über der Zeit t. Dabei steigt die Innentemperatur der Kammer 9 vom Umgebungsdruck T₀ allmählich auf den Zielwert Tₛₒₗₗ. Da im Falle einer Dampfsterilisation bei Betriebsparametern auf der Sattdampfkurve von Wasserdampf der Druck exakt zur Temperatur korrespondiert, kann der Temperaturwert zur Prozessbewertung genutzt werden. Dies bedeutet, dass ein Sensor eines Disposables, welches nicht lange genug der vorgeschriebenen Mindesttemperatur ausgesetzt worden ist, als nicht steril identifiziert und ausgeschleust werden kann. Auf diese Weise kann jedes Disposable auf einfache Weise individuell eine Sterilitätsbescheinigung erhalten. Dies ist im vorliegenden Beispiel auch ohne Temperaturmessung bei alleiniger Druckmessung möglich, wie für dem Fachmann erkennbar ist.

## Patentansprüche

1. Verfahren zum Kalibrieren wenigstens eines Sensors (1), welcher eine Speichereinrichtung zum Speichern wenigstens eines Wertes wenigstens eines Parameters aufweist, mit dem Schritt
Anordnen des Sensors (1) in einer Kammer (9), in welcher zu wenigstens einem Zeitpunkt ein bekannter Wert des Parameters herrscht,
**gekennzeichnet dadurch, dass**
die Kammer (9) eine Sterilisationskammer ist, und dass der Sensor (1) während eines Sterilisationsprozesses in dieser angeordnet ist oder wird,
wobei wenigstens ein während des Sterilisationsprozesses innerhalb der Sterilisationskammer herrschender Wert des wenigstens einen Parameters zu wenigstens einem Zeitpunkt in der Speichereinrichtung des Sensors gespeichert wird.

2. Verfahren nach Anspruch 1, wobei der Sensor (1) ein RFID-Sensor ist.

3. Verfahren nach einem der vorangegangenen Ansprüche, wobei der Parameter ein physikalischer oder chemischer oder biologischer Parameter ist.

4. Verfahren nach einem der vorangegangenen Ansprüche, wobei der Parameter ein Druck, eine Temperatur, eine Konzentration oder eine Leitfähigkeit ist.

5. Verfahren nach einem der vorangegangenen Ansprüche, wobei in der Speichereinrichtung zusätzlich eine Zeitdauer gespeichert wird.

6. Verfahren nach einem der vorangegangenen Ansprüche, wobei eine Mehrzahl von Sensoren (1) gemeinsam in der Kammer (9) angeordnet wird.

7. Verfahren nach Anspruch 6, wobei die Mehrzahl der Sensoren (1) ein Batch von Sensoren (1) ist.

8. Verfahren nach einem der vorangegangenen Ansprüche, wobei der Sensor (1) mit einem Disposable (3) verbunden in der Kammer (9) angeordnet wird.

9. Sensor (1),
**dadurch gekennzeichnet, dass**
er mittels des Verfahrens nach einem der Ansprüche 1 bis 8 kalibriert wurde.

10. Sensor (1) nach Anspruch 9, wobei der Sensor (1) ein RFID-Sensor ist.

11. Disposable (3),
**gekennzeichnet durch**
wenigstens einen Sensor (1) nach Anspruch 9 oder 10.

12. Behandlungsvorrichtung,
**gekennzeichnet durch**
wenigstens einen Sensor (1) nach Anspruch 9 oder 10 und/ oder ein Disposable (3) nach Anspruch 11.

13. Behandlungsvorrichtung nach Anspruch 12, wobei die Behandlungsvorrichtung eine Dialysevorrichtung ist.

## Claims

1. A method for calibrating at least one sensor (1) which comprises a memory means for storing at least one value of at least one parameter, with the step of
placing the sensor (1) inside an enclosure (9) wherein a known value of said parameter prevails at least at one point of time.
**characterized in that**
the enclosure (9) is a sterilization chamber and that the sensor (1) is placed or is being placed in it during a sterilization process,
wherein at least one value of the at least one parameter which prevails in the sterilization chamber during the sterilization process is stored in the memory means of the sensor at least at one point of time.

2. The method according to claim 1, wherein the sensor (1) is an RFID sensor.

3. The method according to one of the preceding claims, wherein the parameter is a physical or chemical or biological parameter.

4. The method according to one of the preceding claims, wherein the parameter is a pressure, a temperature, a concentration, or a conductivity.

5. The method according to any one of the preceding claims, wherein a time period is additionally stored in the memory means.

6. The method according to any one of the preceding claims, wherein a plurality of sensors (1), in particular a batch of sensors (1), is jointly placed in the enclosure (9).

7. The method according to claim 6, wherein the plurality of sensors (1) is a batch of sensors (1).

8. The method according to any one of the preceding claims, wherein the sensor (1) is placed inside the enclosure (9) while being coupled to a disposable (3).

9. A sensor (1),
**characterized by**
having been calibrated by means of the method according to any one of claims 1 through 8.

10. The sensor (1) according to claim 9, wherein the sensor (1) is an RFID sensor.

11. A disposable (3),
**characterized by**
at least one sensor (1) according to claim 9 or 10.

12. A treatment device,
**characterized by**
at least one sensor (1) according to claim 9 or 10 and/or a disposable (3) according to claim 11.

13. The treatment device according to claim 12, wherein the treatment device is a dialysis apparatus.

## Revendications

1. Procédé de calibrage au moins d'un capteur (1), présentant un dispositif de mémorisation permettant de stocker au moins une valeur d'au moins un paramètre, comprenant la phase suivante :
disposition du capteur (1) dans une chambre (9), dans laquelle on trouve une valeur connue du paramètre au moins à un instant donné, **caractérisé en ce que** la chambre (9) est une chambre de stérilisation et **en ce que** le capteur (1) est disposé ou bien va être disposé dans celle-ci pendant un processus de stérilisation, au moins une valeur présente pendant le processus de stérilisation à l'intérieur de la chambre de stérilisation et correspondant à au moins un paramètre à au moins un instant donné est mémorisée dans le dispositif de mémorisation du capteur.

2. Procédé selon la revendication 1, où le capteur est un capteur RFID.

3. Procédé selon l'une des revendications précédentes, où le paramètre est un paramètre physique, chimiques ou biologique.

4. Procédé selon l'une des revendications précédentes, où le paramètre est une pression, une température, une concentration ou une conductivité.

5. Procédé selon l'une des revendications précédentes, où une période est mémorisée en outre dans le dispositif de mémorisation.

6. Procédé selon l'une des revendications précédentes, où une pluralité de capteurs (1) est regroupée dans la chambre (9).

7. Procédé selon la revendication 6, où la pluralité des capteurs (1) est un lot de capteurs (1).

8. Procédé selon l'une des revendications précédentes, où la capteur (1), relié à un élément jetable (3), est disposé dans la chambre (9).

9. Capteur (1), **caractérisé en ce qu'**il a été calibré à l'aide du procédé selon l'une quelconque des revendications 1 à 8.

10. Capteur (1) selon la revendication 9, où le capteur (1) est un capteur RFID.

11. Elément jetable (3), **caractérisé par** au moins un capteur (1) selon la revendication 9 ou 10.

12. Equipement de traitement, **caractérisé par** au moins un capteur (1) selon la revendication 9 ou 10 et/ou un élément jetable (3) selon la revendication 11.

13. Equipement de traitement selon la revendication 12, où l'équipement de traitement est un équipement de dialyse.
